# EUROPÄISCHE PATENTANMELDUNG

(11) **EP 4 685 130 A1**
(43) Veröffentlichungstag der Anmeldung: **28.01.2026**
(21) Anmeldenummer: 24191091.8
(22) Anmeldetag: 26.07.2024
(51) Int. Cl.: C07C 209/68, C07C 211/36

(54) **VERFAHREN ZUR HERSTELLUNG VON METHYLENBIS(CYCLOHEXYLAMIN)**

(71) Anmelder: Evonik Operations GmbH, 45128 Essen (DE)
(72) Erfinder: HILLER, Christoph, 48249 Dülmen (DE); HARDING, Laura-Selin, 46286 Dorsten (DE); NEUKÄUFER, Johannes, 45739 Oer-Erkenschwick (DE); RÖSSLER, Hendrik, 40472 Düsseldorf (DE); VARGAS GÓMEZ, Maria, 45721 Hältern am See (DE); BOERCK, Florian, 58455 Witten (DE); WINKLER, Tobias, 48249 Dülmen (DE); HENGSTERMANN, Axel, 48308 Senden (DE)
(74) Vertreter: Evonik Patent Association

(57) **Zusammenfassung**

Gegenstand der vorliegenden Erfindung ist ein Verfahren zur Herstellung von Methylenbis(cyclohexylamin) umfassend 1) katalytische Hydrierung von MDA, 2) Abtrennung des Katalysators und 3) und nachfolgende Destillation des Produktes der Hydrierung, bei dem in Schritt 3) mindestens eine Trennwandkolonne eingesetzt wird.

## Beschreibung

Die vorliegende Erfindung betrifft ein Verfahren zur Herstellung von Methylenbis(cyclohexylamin).

Methylenbis(cyclohexylamin) ist eine bedeutende Industriechemikalie, die bei vielen typischen Aminreaktionen eingesetzt wird, wie Reaktionen mit Carboxylsäuren, Phosgen, Aldehyden, Ketonen und Epoxiden. Mit Methylenbis(cyclohexylamin) können die Vorteile cycloaliphatischer Amine in Epoxysystemen Anwendung finden: niedrige Mischviskositäten, moderate Reaktivität und wenig exothermes Verhalten sowie herausragende mechanische Eigenschaften und exzellente Chemikalienbeständigkeit. Im Vergleich zu anderen Aminen ist die Neigung zur Carbamatbildung vermindert, was einen Vorteil für die Verwendung als Epoxidhärter darstellt.

Methylenbis(cyclohexylamin) ist ein cycloaliphatisches Amin, das üblicherweise über Hydrierung von Diaminodiphenylmethan hergestellt wird. Diaminodiphenylmethan wird aufgrund dessen wiederum aus Anilin und Formaldehyd erfolgender Herstellung auch als Methylendianilin bezeichnet. Als Abkürzung für Methylendianilin wird für Diaminodiphenylmethan auch oft die Bezeichnung MDA verwendet. Entsprechend wird Methylenbis(cyclohexylamin) auch oft als H12MDA bezeichnet.

MDA ist aufgrund seines Herstellungsprozesses ein Gemisch aus verschiedenen Diaminodiphenylmethanen - vor allem 4,4`-Diaminodiphenylmethan, es können jedoch auch 2,4`- und 2,2'-lsomere vorliegen. Weiterhin können in dem Gemisch Reaktionsprodukte mit drei oder mehr aromatischen Ringen, insbesondere solche mit drei oder mehr Phenylringen, vorliegen. Die entsprechenden Reaktionsprodukte mit drei oder mehr aromatischen Ringen werden auch als Mehrkernverbindungen bezeichnet.

Bei dem im Handel erhältlichen Methylenbis(cyclohexylamin) handelt es sich aufgrund der Zusammensetzung des eingesetzten MDAs größtenteils um 4,4'-Diaminodicyclohexylmethan bzw. Bis(para-Aminocyclohexyl)methan. 4,4`-Diaminodicyclohexylmethan kann wiederum als trans/trans-, cis/cis- und cis/trans-Isomer vorliegen und ist deswegen in der Regel ein Gemisch dieser Isomere mit unterschiedlichen Anteilen. Während Methylenbis(cyclohexylamin)-Qualitäten mit niedrigem trans/trans-Gehalt an 4,4'-Diaminodicyclohexylmethan (10-30 %) im Bereich der Amin- und Isocyanat-Vernetzer, insbesondere im Bereich von 2-Komponenten-Harzen, Einsatz finden, finden Qualitäten mit hohem trans/trans-Gehalt (> 48 %) vor allem Anwendung als Regulator in Polyamidverbindungen. Aufgrund der potentiellen Anwesenheit der entsprechenden 2,4'- und 2,2'-Diaminophenylmethan-lsomere im MDA kann in Methylenbis(cyclohexylamin) auch 2,4`-Diaminodicyclohexylmethan und 2,2'-Diaminodicyclohexylmethan vorliegen. Weiterhin kann Methylenbis(cyclohexylamin) weiterhin noch (ggf. partiell) hydrierte Mehrkernverbindungen enthalten.

Es gibt Stand der Technik, in dem Methylenbis(cyclohexylamin) nicht das gewünschte Hauptprodukt einer Synthese ist und von diesem abgetrennt wird.

US 2,511,028 A offenbart die partielle Hydrierung von Bis(4-Aminophenyl)methan unter Erhalt des entsprechenden Produktes mit nur noch einem aromatischen Ring. Die Abtrennung von Methylenbis(cyclohexylamin) vom gewünschten Hauptprodukt kann destillativ erfolgen.

US 4,399,307 A befasst sich mit der destillativen Abtrennung von Nebenprodukten von einem Produkt, das Methylenbis(cyclohexylamin) strukturell ähnelt, nämlich dem Diamin 2,2`-bis(4-Aminocyclohexyl)propan (PACP). Es wird ein Verfahren zur Abtrennung einer Gruppe organischer Verunreinigungen, die Bis(4-Aminocyclohexyl)methan einschließt, durch Zugabe eines Lösemittels und Destillation offenbart.

Verfahren zur Herstellung von Methylenbis(cyclohexylamin) sind seit langem im Stand der Technik bekannt.

So offenbart US 3,742,049 A ein Verfahren zur Herstellung eines Bis(4-Aminocyclohexyl)methans bzw. - ethans, bei dem das entsprechende Bis(4-nitrophenyl)alkan in Gegenwart eines Rutheniumoxid-Katalysators hydriert wird. Die Herstellung der Edukte und ihr Handling ist jedoch aufwändig.

Ebenfalls seit langem im Stand der Technik offenbart sind Verfahren zur Herstellung von Bis(4-Aminocyclohexyl)methan aus MDA.

EP 0 639 403 A2 offenbart ein Verfahren zur Hydrierung von MDA in der Schmelze. Eine Aufreinigung des Produktes wird nicht beschrieben.

Verfahren zur Hydrierung von MDA in Lösung gehören ebenfalls seit langem zum Stand der Technik: US 5,214,212 A offenbart ein Verfahren zur katalytischen Hydrierung von MDA zu Bis(4-Aminocyclohexyl)methan, bei dem neben Wasserstoff ein Edelmetallkatalysator, ein Promoter und ein Lösemittel eingesetzt wird. In den Beispielen wird das Produkt aus dem Reaktionsgemisch über Filtration abgetrennt.

US 2002/0183556 A1 offenbart die Hydrierung von MDA zu Methylenbis(cyclohexylamin) in einem Suspensionsreaktor, bei dem ein Rutheniumkatalysator eingesetzt wird. Das optional vorliegende Lösemittel kann destillativ abgetrennt werden.

Auch DE 101 19 135 A1 offenbart ein Verfahren zur Herstellung von MDA zu Methylenbis-(cyclohexylamin) in einem Suspensionsreaktor. Dieser kann aus einer Kaskade mehrerer verschalteter Reaktoren bestehen. Das optional vorliegende Lösemittel kann destillativ abgetrennt werden.

EP 1 566 372 A1 offenbart ein Verfahren zur Hydrierung einer Substanz in einem Rieselbettreaktor. Bei der eingesetzten Substanz kann es sich um MDA handeln.

US 2005/0261525 A1 offenbart ein Verfahren zur katalytischen Hydrierung von MDA, bei dem ein Lithiumaluminat-geträgerter Rhodium- und Ruthenium-haltiger Katalysator eingesetzt wird.

EP 3 000 803 A1 offenbart ein Verfahren zur Herstellung von Diaminodicyclohexylmethan, bei dem zunächst 4,4`-Diaminophenylmethan hydriert wird, bis die Umwandlung 90-98 % beträgt. Dann wird statt reinem 4,4`-Diaminophenylmethan ein Gemisch von 2,4`-Diaminophenylmethan und 4,4'-Diaminophenylmethan zugeführt. Nachdem die Umwandlung dieses Gemisches mehr als 90 % beträgt, wird die Umwandlung mehrere Stunden fortgeführt. Danach wird wieder ausschließlich 4,4`-Diaminophenylmethan zugeführt.

Im Stand der Technik sind weiterhin Verfahren bekannt, bei denen 4,4`-Diaminodicyclohexylmethan hergestellt und im Anschluss destillativ aufgearbeitet wird, um sauberere Produkte zu erhalten:
DE 25 02 893 A1 offenbart ein Verfahren zur Herstellung cycloaliphatischer Amine durch katalytische Hydrierung der entsprechenden aromatischen Amine in Gegenwart eine Rutheniumkatalysators. In den Beispielen wird 4,4'-Diaminodiphenylmethan in Gegenwart eines Katalysators hydriert. Das Reaktionsprodukt wird in Methanol aufgenommen, filtriert und destilliert.

US 3,856,862 A offenbart ein Verfahren zur katalytischen Hydrierung von 4,4`-Diaminodiphenylmethan zu 4,4`-Diaminodicyclohexylmethan in Gegenwart von Ammoniak, bei dem ein geträgerter RhodiumKatalysator eingesetzt wird. In den Beispielen wird erhaltenes Methylenbis(cyclohexylamin) nach Abtrennung des Katalysators, Waschen mit Isopropanol und Abziehen des Lösemittels durch Einsatz einer micro-Vigreux-Kolonne aufgereinigt. Das Produkt enthält größtenteils Methylenbis(cyclohexylamin) mit geringen Anteilen des ebenfalls anfallenden partiellen Hydrierproduktes H6MDA, das nur einen Cyclohexylring aufweist, und andere Verunreinigungen.

US 4,754,070 A offenbart ein Verfahren zur katalytischen Hydrierung von 4,4`-Diaminodiphenylmethan mit einem Oligomergehalt von 10 - 30 % in Gegenwart eines Katalysators, der die Metalle Rhodium und Ruthenium umfasst. In einer Variante des Beispiels wird das lösemittelhaltige Produktgemisch destillativ aufgetrennt.

WO 2006/065961 A1 offenbart ein Verfahren zur gemeinsamen Hydrierung von MDA und mindestens einem zweiten aromatischen Amin. In den Beispielen wird ein 1 : 1-Gemisch von MDA und Anilin in Gegenwart eines Ruthenium/Aluminiumoxid-Katalysators zu Diaminodicyclohexylmethan und Cyclohexylamin umgesetzt. Das Reaktionsgemisch wird fraktioniert destilliert.

WO 2009/090179 A2 offenbart ein Verfahren zur Herstellung von cycloaliphatischen Aminen durch Hydrierung der entsprechenden aromatischen Verbindungen mit Wasserstoff enthaltendem Gas in Gegenwart von Ruthenium-haltigen Katalysatoren und in Anwesenheit suspendierter anorganischer Zusatzstoffe. Bei der eingesetzten aromatischen Verbindung kann es sich um 4,4`-Diaminodiphenylmethan handeln. Das nach der Reaktion erhaltene Gemisch wird kann über Rektifikation oder Destillation aufgereinigt werden.

WO 2009/144148 A1 offenbart ein Verfahren zur Herstellung aromatischer Diisocyanate, bei dem in einem Teilschritt aromatische Amine in Gegenwart eines Katalysators zu cycloaliphatischen Aminen hydriert werden. Beispiel 2 setzt als aromatisches Amin MDA ein. Das Produkt wird destillativ aufgereinigt.

WO 2009/153123 A1 offenbart ein Verfahren zur Hydrierung organischer Verbindungen in einem mehrphasigen System in Gegenwart eines homogenen oder heterogenen Katalysators, das in zwei Stufen durchgeführt wird. Ein bevorzugt einsetzbares Edukt ist MDA. Gemäß Beschreibung kann die Reinigung des erhaltenen Reaktionsgemisches über Destillation erfolgen.

EP 2 883 863 A1 offenbart ein Verfahren zur Hydrierung von 4,4'-Methylendianilin mit Wasserstoff in Gegenwart eines Katalysators, der Ruthenium auf einem Zirkoniumdioxid-Trägermaterial enthält. Die erhaltenen Hydriergemische können nach dem erfindungsgemäßen Verfahren gereinigt werden, beispielsweise durch Destillation.

EP 2 502 900 A1 offenbart ein Verfahren zur Herstellung von 4,4'-Diaminocyclohexylmethan, bei dem MDA in Gegenwart eines organischen Lösemittels und eines Katalysators hydriert wird und die Reaktion gestoppt wird, wenn die Reaktionslösung 0 - 5 Gew.-% MDA und 1 - 20 Gew.-% H6MDA aufweist. Nachfolgend wird erhaltenes 4,4'-Diaminocyclohexylmethan isoliert. In einer Ausführungsform wird das Reaktionsgemisch filtriert, um den Katalysator zu recyclieren und ihn erneut der Reaktion zuzuführen. Das Filtrat wird in einer ersten Destillationskolonne destilliert, und das Lösemittel am Kopf der Kolonne entnommen, während ein Roh-H12MDA-Strom am Boden der Kolonne entnommen wird. Der Roh-H12MDA-Strom wird in einer zweiten Kolonne destillativ aufgereinigt, wobei Leichtsieder/nicht aminierte Produkte am Kopf der Kolonne entnommen werden und Produktstrom am Boden der Kolonne entnommen wird. Schließlich wird der am unteren Ende der zweiten Kolonne entnommene Produktstrom einer dritten Destillationskolonne zugeführt und so destilliert, dass 4,4'-Diaminocyclohexylmethan am Kopf der Kolonne entnommen wird, MDA und H6MDA seitlich entnommen (und recycliert) werden können und sekundäre Amine/Hochsieder am Boden der Kolonne verbleiben.

Die Nachteile der vorgenannten Verfahren und insbesondere gegenüber EP 2 502 900 A1 sind eine höhere Temperaturbelastung durch die konstruktiv bedingte Ausführung mit zwei Kolonnen für die Auftrennung des Produktes und eine damit verbundene höhere Verweilzeit. Nachteilig ist weiterhin bei den vorgenannten Verfahren, dass in der zweiten Kolonne das Methylenbis(cyclohexylamin) am Kopf kondensiert wird. Durch den höheren Schmelzpunkt bestimmter Isomere des Methylenbis(cyclohexylamin)s kristallisieren diese zum Teil aus, was eine zeitweilige Außerbetriebnahme der Kolonne erfordert und somit die Betriebszeiten verringert oder alternativ den Einsatz von zwei parallel geschalteten Kondensatoren erfordert, was jedoch einen hohen Aufwand darstellt und Probleme bei der Betreibbarkeit erzeugt.

Schließlich wäre es weiterhin wünschenswert, wenn das Isomerenverhältnis des Methylenbis(cyclohexylamin)s bei der Destillation konstant bliebe.

Aufgabe der vorliegenden Erfindung ist es somit, die Nachteile des Standes der Technik zu überwinden. Insbesondere ist es Aufgabe der vorliegenden Erfindung, ein Verfahren zur Herstellung und Aufreinigung von Methylenbis(cyclohexylamin) über Destillation bereitzustellen, bei dem die Temperaturbelastung und die Verweilzeit des Produktes vermindert ist und Probleme durch Kristallisation nicht auftreten. Weiterhin ist es die Aufgabe der vorliegenden Erfindung, dass das Isomerenverhältnis des Methylenbis(cyclohexylamin)s bei der Destillation weitestgehend konstant bleibt.

Diese sich vorliegend stellende Aufgabe wird gelöst durch das erfindungsgemäße Verfahren zur Herstellung von Methylenbis(cyclohexylamin) umfassend
1) katalytische Hydrierung von MDA,
2) Abtrennung des Katalysators und
3) nachfolgende Destillation des Produktes der Hydrierung,
wobei bei der Destillation in Schritt 3) mindestens eine Trennwandkolonne eingesetzt wird.

Vorteilhafterweise liegt der Energieverbrauch für das erfindungsgemäße Trennverfahren bei 450 bis 500 Kilojoule pro Kilogramm Methylenbis(cyclohexylamin). Die benötigte Energiemenge bei einer Zwei-Kolonnenverschaltung beträgt dagegen zwischen 550 bis 650 Kilojoule pro Kilogramm Methylenbis(cyclohexylamin).

### Schritt 1) - katalytische Hydrierung

Schritt 1) ist die katalytische Hydrierung von MDA zu Methylenbis(cyclohexylamin) in Gegenwart eines Katalysators.

Als Katalysatoren können prinzipiell alle Verbindungen eingesetzt werden, die die Hydrierung von Phenylgruppen katalysieren. Prinzipiell können dies homogene oder heterogene Katalysatoren sein. Bevorzugt sind jedoch heterogene Katalysatoren.

Insbesondere haben sich Katalysatoren umfassend Aktivmetalle ausgewählt aus Nickel, Cobalt, Palladium, Platin, Ruthenium und/oder Rhodium als besonders geeignet erwiesen.

Zur Erhöhung der Aktivität, Selektivität und/oder Standzeit können die Katalysatoren zusätzlich Dotiermetalle oder Modifizierungsmittel enthalten oder mit diesen behandelt worden sein. Bevorzugte Dotiermetalle können ausgewählt werden aus der Gruppe bestehend aus Mo, Fe, Ag, Cr, V, Ga, In, Bi, Ti, Zr, Mn und den seltenen Erden. Bevorzugte Modifizierungsmittel sind solche, mit denen die Säure-Base-Eigenschaften der Katalysatoren beeinflusst werden können, insbesondere Alkalimetalle, Erdalkalimetalle, Phosphorsäure und Schwefelsäure sowie deren Verbindungen bzw. Salze.

Die Katalysatoren können bevorzugt in Form von Pulvern oder Formkörpern, wie z. B. Extrudaten oder gepressten Pulvern, eingesetzt werden. Es können Vollkontakte, Raney-Typ-Katalysatoren oder Trägerkatalysatoren zur Anwendung kommen.

Bevorzugte Trägermaterialien für Trägerkatalysatoren sind Aktivkohle und anorganische Oxide, insbesondere Al₂O₃, SiO₂, TiOz, ZrOz, ZnO und MgO, sowie weiterhin Bentonite, Alumosilikate, Kaoline, Tone, Kieselgure und Lithiumaluminate. Das Aktivmetall kann in einer dem Fachmann bekannten Weise auf das Trägermaterial aufgebracht werden, z. B. durch Imprägnierung, Aufsprühen oder Fällung. Je nach Art der Katalysatorherstellung sind weitere, dem Fachmann bekannte Präparationsschritte notwendig, wie z. B. Trocknung, Calcinierung, Formgebung und Aktivierung. Zur Formgebung können optional weitere Hilfsstoffe wie z. B. Graphit oder Magnesiumstearat zugesetzt werden.

Bevorzugt eingesetzt werden Trägerkatalysatoren mit Ruthenium, Rhodium oder Rh/Ru-Kombinationen als wesentlichen Aktivmetallen. Bevorzugte Trägermaterialien sind solche auf Basis von Al₂O₃, SiO₂, TiOz und ZrOz Ganz besonders bevorzugt sind Al₂O₃ und SiO₂.

Bevorzugt werden solche Katalysatoren eingesetzt, von denen bekannt ist, dass mit ihnen ein Methylenbis(cyclohexylamin) mit einem trans/trans-Anteil des 4,4'-lsomeren zwischen 10 und 30 %, insbesondere zwischen 15 und 25 % hergestellt werden kann. Solche Katalysatoren sind beispielsweise in den Dokumenten EP 1 366 812 A1, EP 0 066 211 A1, DE 100 54 347 A1, EP 0 392 435 A1,

EP 0 630 882 A1, EP 0 639 403 A2 und US 5,545,756 A beschrieben. Ganz besonders bevorzugt wird die Hydrierung in Gegenwart eines geträgerten Katalysators durchgeführt, der Aktivmetall in einer Menge von 0,01 bis 20 Gew.-%, bezogen auf den geträgerten Katalysator, auf einen Träger aufgebracht enthält, und dessen Aktivmetall Ruthenium alleine oder Ruthenium und mindestens ein Metall der I., VII. oder VIII. Nebengruppe des Periodensystems ist. Mit diesem Katalysator können besonders niedrige trans/trans-Gehalte von 4,4'-Diamino-dicyclohexylmethan erzielt werden.

Um gezielt und reproduzierbar ein Methylenbis(cyclohexylamin) mit einem bestimmten trans/trans-Anteil des 4,4'-lsomeren herstellen zu können, ist es weiterhin vorteilhaft, die Temperatur, den Umsatz und die Verweilzeit im Reaktor genau zu kontrollieren. Aus diesem Grund wird die Hydrierung bevorzugt in kontinuierlich betriebenen Reaktoren durchgeführt, da diese eine entsprechende Kontrolle ermöglichen. Geeignete Reaktoren für die kontinuierliche Hydrierung sind dem Fachmann geläufig. In einer bevorzugten Ausführungsform der Erfindung wird die kontinuierliche Hydrierung des MDA in Festbettreaktoren durchgeführt.

Bevorzugt wird die Reaktion in zwei oder mehr in Reihe geschalteten Reaktionsräumen durchgeführt. Der Vorteil dieser Reaktionsführung besteht vor allem darin, dass die Reaktionsräume unabhängig voneinander geheizt oder gekühlt werden können und sich dadurch bessere Steuerungsmöglichkeiten des trans/trans-4,4'-H12MDA-Anteils ergeben. Ein weiterer Vorteil besteht darin, dass ein Nachlassen der Katalysatoraktivität gezielter durch eine Temperaturanpassung ausgeglichen werden kann und bei Bedarf partielle Katalysatorwechsel möglich sind. Die getrennten Reaktionsräume können z. B. durch zwei oder mehr in Reihe geschaltete Festbettreaktoren, wie z. B. Rohrbündelreaktoren und/oder Schachtöfen, realisiert werden. Eine andere Möglichkeit besteht darin, in einem Reaktor räumlich voneinander getrennte Katalysatorschüttungen unterzubringen, die geheizt oder gekühlt werden können. Die Festbettreaktoren können in Sumpffahrweise betrieben werden, bevorzugt wird aber eine Rieselbettfahrweise. Bevorzugte kontinuierliche Suspensionsreaktoren sind Schlauch- und Blasensäulenreaktoren.

Der LHSV-Wert liegt bevorzugt im Bereich von 0,01 bis 1 h⁻¹ (I des zu hydrierenden aromatischen Amins pro I Festbettkatalysator und Stunde).

Die Hydrierung erfolgt weiterhin bevorzugt bei Temperaturen im Bereich von 50 bis 200 °C, vorzugsweise zwischen 80 und 170 °C. Der Wasserstoffdruck liegt dabei bevorzugt zwischen 1 und 30 MPa, bevorzugt zwischen 5 und 15 MPa.

Prinzipiell kann bei der Hydrierung ein Lösemittel anwesend sein, es muss es aber nicht. Bevorzugt wird jedoch MDA in einem Lösungsmittel hydriert. Der Anteil des Lösungsmittels liegt weiter bevorzugt zwischen 10 und 90 %, noch weiter bevorzugt zwischen 50 und 90 % bezogen auf die Masse der Lösung. Bevorzugte Lösungsmittel können ausgewählt werden aus der Gruppe bestehend aus primären, sekundären und tertiären ein- oder mehrwertigen Alkoholen (insbesondere Methanol, Ethanol, n- und i-Propanol, 1-, 2-, i- und tert.-Butanol, Ethylenglykol, und Ethylenglykolmono(C1-C3)alkylether), linearen Ethern (insbesondere Ethylenglykoldi(C1-C3)alkylether) und MTBE, cyclischen Ethern (insbesondere Tetrahydrofuran und Dioxan) und Alkanen (insbesondere n- und iso-Alkane mit 4-12 C-Atomen, weiter bevorzugt n-Pentan, n-Hexan und Isooctan, und cyklischen Alkanen, weiter bevorzugt Cyclohexan und Dekalin). Während Alkohole zu einer Alkylierung der Aminogruppen führen können, weisen Ether diesen Nachteil nicht auf und sind somit besonders bevorzugt. Ganz besonders bevorzugtes Lösungsmittel ist Tetrahydrofuran.

Lösungsmittel kann jedoch auch ebenfalls bevorzugt das Hydrierprodukt selbst sein.

Die Hydrierung kann bevorzugt auch in Gegenwart von Ammoniak, einem primären, sekundären oder tertiären Amin oder einem polycyklischen Amins mit einem verbrückendem N-Atom durchgeführt werden.

Besonders bevorzugt wird als zu hydrierendes Stoffgemisch ein MDA eingesetzt, das mindestens 70 Gew.-% 4,4'-Diaminodiphenylmethan und 0,01 bis 2 Gew.-% N-Methylverbindungen, jeweils bezogen auf die Gesamtmasse an Verbindungen mit aromatischen Ringen, aufweist. Noch weiter bevorzugt wird ein MDA bestehend aus 74 - 85 Gew.-% 4,4'-MDA, 3 - 20 Gew.-% 2,4'-MDA, weniger als 1 Gew.-% 2,2'-MDA und bis zu 1 Gew. % N-Methylverbindungen eingesetzt. Mit den genannten Gemischen kann besonders gut ein Hydriergemisch erhalten werden, das einen trans/trans-Anteil des 4,4'-lsomeren von 10 bis 30 % aufweist.

### Schritt 2) - Abtrennung des Katalysators

In Schritt 2) wird der Katalysator abgetrennt. Handelt es sich bei dem Katalysator um einen Festbettkatalysator, geschieht dies dadurch, dass das hydrierte Produkt aus dem Festbettreaktor ausgeschleust wird. Handelt es sich bei dem Katalysator um einen heterogenen Katalysator, der kein Festbettkatalysator ist, kann dies bevorzugt über Dekantieren, Zentrifugieren oder Filtration erfolgen. Besonders bevorzugt erfolgt die Abtrennung über Filtration, wobei der Einsatz von Siebfiltern, Kerzenfiltern oder Cross-Flow-Filtern ganz besonders bevorzugt ist.

Weiterhin kann vor oder nach der Abtrennung des Katalysators das oder die Lösemittel, sofern eingesetzt, entfernt werden. Weiter bevorzugt wird das oder die Lösemittel entfernt, nachdem der Katalysator abgetrennt wurde.

Bevorzugt wird das Lösemittel über Destillation entfernt. Weiter bevorzugt kann dies durch Verwendung einer Destillationskolonne geschehen. Um eine Akkumulation des/der Lösemittel im Folgenden weiter zu vermindern, kann weiterhin ein Purge-Schritt vorgesehen werden.

### Schritt 3) - Destillation des Produktes der Hydrierung

Nach der Abtrennung von Katalysator und ggf. Lösemittel wird das erhaltene Rohprodukt der Hydrierung destilliert. Dabei wird mindestens eine Trennwandkolonne eingesetzt.

Die Destillation des Methylenbis(cyclohexylamin) umfassenden Produktes der Hydrierung kann somit eine, zwei oder mehr als zwei Trennwandkolonnen vorsehen.

Bevorzugt erfolgt die Destillation des Produktes der Hydrierung jedoch ausschließlich durch Einsatz einer Trennwandkolonne, da so eine besonders niedrige Verweilzeit des Produktes in der Destillation bei dennoch ausreichender Auftrennung gewährleistet wird und das energetische Optimum erreicht wird.

Weiterhin kann vor oder nach der Destillation des Produktes der Hydrierung mittels mindestens einer Trennwandkolonne noch mindestens eine Kolonne, die keine Trennwandkolonne ist, zur destillativen Auftrennung vorgesehen sein. Bevorzugt wird auf diese jedoch verzichtet, da die Trennwirkung der mindestens einen Trennwandkolonne bereits genügt.

Weiter bevorzugt wird die mindestens eine Trennwandkolonne so betrieben, dass das Produkt Methylenbis(cyclohexylamin) an der Seite der Trennwandkolonne ausgetragen wird. So wird besonders effizient eine Auskristallisation vermieden, da das Produkt nicht mit dem Kondensator in Kontakt gelangt. Die Entnahme erfolgt bevorzugt in flüssiger Form.

Ganz besonders bevorzugt erfolgt die Destillation des Produktes der Hydrierung in Schritt 3) ausschließlich durch Einsatz einer Trennwandkolonne.

Die Konstruktion von Trennwandkolonnen ist dem Fachmann bekannt. Diese weisen im Gegensatz zu anderen Kolonnen eine in der Kolonne befindliche, weitestgehend vertikal angeordnete Trennwand auf.

Dabei kann sich die Trennwand prinzipiell an beliebiger Position innerhalb der Trennwandkolonne befinden. So ist es zum Beispiel möglich, dass i) sich die Trennwand von einem trennwandlosen Sumpfbereich bis zu einem trennwandlosen Kopfbereich erstreckt, dass ii) die Trennwand das untere Ende der Kolonne verschließt, aber nicht den Kopfbereich oder dass iii) die Trennwand den Kopfbereich verschließt, aber nicht das untere Ende der Kolonne.

Bevorzugt erstreckt sich die Trennwand von einem trennwandlosen Sumpfbereich bis zu einem trennwandlosen Kopfbereich. Noch weiter bevorzugt befindet sich die Trennwand der mindestens einen Trennwandkolonne, bevorzugt der Trennwandkolonne, durchgehend in einer Höhe von 15 bis 80 %, weiter bevorzugt in einer Höhe von 20 bis 75 %, besonders bevorzugt in einer Höhe von 25 bis 75 %, der Trennwandkolonne, gemessen vom unteren Ende derselben. Als "Höhe" der Trennwandkolonne ist dabei der Abstand von Kolonnenboden bis zum Kolonnenkopf zu verstehen.

Üblicherweise befindet sich die Trennwand mittig in der Kolonne. Die Trennwand kann jedoch auch zur Erzielung vorteilhafter Eigenschaften einen Versatz in Richtung des Zulaufs oder in Richtung der Seitenstromentnahme aufweisen. Bevorzugt beträgt der Versatz der Trennwand einen Wert zwischen 0 und 30 % des Durchmessers von der Mitte in Richtung des Seitenstrombereichs oder des Zulaufbereichs. Weiter bevorzugt beträgt dieser Wert zwischen 0 und 20 %.

Die Trennwand kann zur Erzielung vorteilhafter Ergebnisse auch als asymmetrische Trennwand ausgestaltet sein. Eine asymmetrisch ausgestaltete Trennwand weist einen horizontalen Versatz auf. Weiter bevorzugt ist hierbei der horizontale Versatz so ausgestaltet, dass sich das untere Ende der Trennwand näher an der Seitenstromentnahme befindet als das obere Ende. Dadurch verringert sich dort die Querschnittfläche unterhalb der Seitenstromentnahme, während sie im oberen Bereich auf der Seite des Zulaufs erhöht ist. Der Vorteil dieses Aufbaus besteht in einer Optimierung der Flüssigkeitsbelastung und der Gasverteilung und damit des Druckverlusts der Kolonne. Weiter bevorzugt beträgt der Versatz der Trennwand oberhalb des Zulaufs 2 bis 30 % der Querschnittsfläche der Kolonne und der Versatz der Trennwand unterhalb des Seitenstrom zwischen 2 und 40 % der Querschnittsfläche der Kolonne. So kann ein besonders positiver Effekt in Bezug auf die Qualitäts-, Ausbeute- und Energieanforderungen erzeugt werden.

Bevorzugt wird die Destillation mit der mindestens einen Trennwandkolonne, weiter bevorzugt der Trennwandkolonne, zur Vermeidung von Kristallisation so betrieben, dass
a) sich auf der Höhe mindestens eines Teils der Trennwand der Zulauf befindet,
b) sich auf der Höhe mindestens eines Teils der Trennwand auf der anderen Seite der Trennwand, bezogen auf den Zulauf, die Seitenstromentnahme zur Entnahme von aufgereinigtem Methylenbis(cyclohexylamin) befindet,
c) am Kopf der Trennwandkolonne das Destillat entnommen, und zum Teil ausgetragen und zum Teil wieder in die Trennwandkolonne zurückgeführt wird und
d) der am Boden der Trennwandkolonne befindliche Sumpf ausgeschleust wird.

Dabei handelt es sich bei dem in Schritt b) entnommenen Methylenbis(cyclohexylamin) um das gewünschte Produkt des Verfahrens. Das in c) entnommene Destillat umfasst Leichtsieder und ggf. noch Lösemittel. Im Sumpf befinden sich Schwersieder, die u. a. Mehrkernverbindungen umfassen können.

Weiter bevorzugt wird die Entnahme des Destillats in Schritt c) so ausgeführt, dass
- am Kopf der Trennwandkolonne das Destillat entnommen,
- es in einem angeschlossenen Kondensator mindestens partiell kondensiert,
- und mindestens ein Teil des Kondensats in den Kopfbereich der Trennwandkolonne zurückgeführt und ein ggf. hinter dem Kondensator noch flüchtig vorliegender Leichtsiederstrom zusammen mit nicht rückgeführtem Kondensat ausgetragen wird.

Bevorzugt befindet sich der Zulauf der mindestens einen Trennwandkolonne, bevorzugt der einen Trennwandkolonne, in einer Höhe von 25 bis 66 %, weiter bevorzugt in einer Höhe von 30 bis 60 %, noch weiter bevorzugt in einer Höhe von 40 bis 60 %, der Höhe der Kolonne, gemessen vom unteren Ende derselben.

Die mindestens eine Trennwandkolonne, bevorzugt die Trennwandkolonne, weist weiterhin bevorzugt eine Zahl theoretischer Trennstufen von 10 bis 90, weiter bevorzugt von 15 bis 80, weiter bevorzugt von 20 bis 70 auf.

Die mindestens eine Trennwandkolonne, bevorzugt die Trennwandkolonne, wird bevorzugt bei einer Sumpftemperatur von 190 bis 300 °C, weiter bevorzugt von 190 bis 270 °C, ganz besonders bevorzugt von 190 bis 240°C, betrieben, da so Produkte unter Erzielung besonders guter Qualitäten (insbesondere mit besonders gering ausgeprägter Farbigkeit) und Ausbeuten und mit besonders geringen Energieanforderungen erzielt werden können. Der Druck beträgt dabei bevorzugt 1 bis 30 mbar, weiter bevorzugt 2 bis 20 mbar und ganz besonders bevorzugt 3 bis 15 mbar.

Die mindestens eine Trennwandkolonne, bevorzugt die Trennwandkolonne, wird bevorzugt mit einer Flüssigkeitsverteilung der oberhalb der Trennwandkolonne herabfließenden Flüssigkeit auf die beiden Seiten in einem Verhältnis zwischen 10/90 und 40/60 (Zulauf-/ Seitenstromteil), bevorzugt zwischen 15/85 und 30/70, betrieben.

Eine besonders gute Trennung des Produktes der Hydrierung, d.h. eine besonders reines Methylenbis(cyclohexylamin), kann erhalten werden mit einer Flüssigkeitsbelastung von kleiner oder gleich 1,5 m³/(m²h) unterhalb der Seitenstromabnahme und oberhalb des Sumpfes und oberhalb des unteren Endes der Trennwand, weiter bevorzugt zwischen 0,1 und 1 m³/(m²h), da die Trennung so am energetischen Optimum betrieben werden kann. Diese niedrige Flüssigkeitsbelastung und die erforderliche Aufteilung auf die beiden Bereiche (Zulaufteil und Seitenstromteil) stellt für den Trennwandkolonnenaufbau einen besonders niedrigen Fall dar, wodurch die Betreibbarkeit zunehmend abnimmt, besonders durch die hydraulische Kopplung der Flüssigkeitsströme einer Trennwandkolonne. Die Flüssigkeit, die oberhalb der Trennwand auf den Seitenstrombereich gegeben wird, wird bei energetisch optimaler Betriebsweise zum Großteil durch den Seitenstrom abgenommen. Wird somit die Flüssigkeitsbelastung unterhalb des Seitenstrom zu niedrig betrieben, können Schwankungen in der Flüssigkeitsaufteilung und der Seitenstromabnahme nicht mehr kompensiert werden und es kommt zu einem Zusammenbruch der Trennleistung.

Neben der geringeren Flüssigkeitsbelastung ist ein sehr niedriger Druckverlust zwischen Kopf und Sumpf der Kolonne wünschenswert, da ein zu hoher Druckverlust zu höheren Drücken im Sumpf und somit zu höheren Siedetemperaturen der im Sumpf vorhandenen Komponenten führt. Sehr hohe Temperaturen im Sumpf wiederum führen zu zwei praktischen Problemen. Zum einen kann bei den Sumpfkomponenten eine Zersetzung ab 255 °C festgestellt werden, welche dann dazu führt, dass die Spezifikation für das Seitenstromprodukt Methylenbis(cyclohexylamin) nicht mehr erreicht werden können. Zum anderen wird die Beheizung des Sumpfs schwieriger, da eine Beheizung mit Dampf nicht mehr möglich ist auf den dann erreichten Temperaturniveaus.

Die Seitenstromentnahme der mindestens einen Trennwandkolonne, bevorzugt der einen Trennwandkolonne, befindet sich bevorzugt in einer Höhe von 30 bis 60 %, weiter bevorzugt in einer Höhe von 40 bis 60 %, noch weiter bevorzugt in einer Höhe von 45 bis 55 %, der Höhe der Kolonne, gemessen vom unteren Ende derselben.

Die Packungsauswahl ist von sehr hoher Bedeutung für die Durchführung der Destillation, da der Druckverlust für diesen Anwendungsfall bevorzugt unter 0,2 mbar pro theoretischer Trennstufe liegen sollte. Die Kombination aus niedriger Flüssigkeitsbelastung, dem niedrigen Druckverlust und den damit verbundenen Eigenschaften, die eine Packung erfüllen muss, lassen vermuten, dass der Einsatz einer Trennwandkolonne nicht zu bevorzugen ist. Überraschend wurde gefunden, dass eine Trennwandkolonne trotz dieser hohen Herausforderungen und der benötigten feinen Methylenbis(cyclohexylamin)-Trennung betrieben werden kann.

Die Packungsbetten der Trennwandkolonne sind bevorzugt zwischen 2 und 7 Metern Höhe ausgelegt. Weiter bevorzugt trennen sie Flüssigkeiten sehr fein auf, sodass es zu einer trenntechnisch akzeptablen Fehlverteilung innerhalb der Packungsbetten kommt.

Als Packungstypen werden bevorzugt geordnete und/oder ungeordnete Packungen, weiter bevorzugt Blech- und/oder Gewebepackungen eingesetzt. Diese erzielen eine besonders hohe Trennleistung bei niedrigem Druckverlust und niedriger Flüssigkeitsbelastung.

Um die Produktqualität und die Validität der Trennwandkolonne für die Trennung von Methylenbis(cyclohexylamin) und den leicht- und schwersiedenden Nebenkomponenten zu zeigen, wurden experimentelle Untersuchungen durchgeführt.

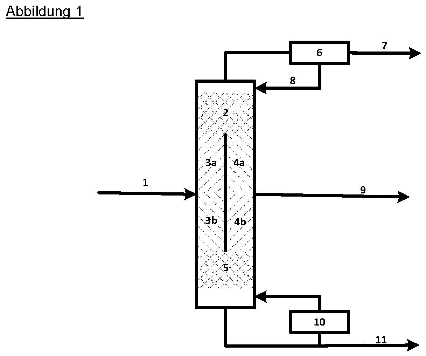

Dabei wurde festgestellt, dass bestimmte Ausführungen der Trennwand positive Effekte erzielen. Die verschiedenen Varianten sind in den Abbildungen 1, 3, 4 und 5 dargestellt sind. Die Ausführungen der Wand in den Abbildungen 1, 3, 4 lassen sich auch in der Patentschrift EP 2 569 274 B1 finden. Im Gegensatz zum Verfahren des genannten Patents zeichnet sich das erfindungsgemäße Verfahren durch einen niedrigeren Betriebsdruck, den daraus resultierenden veränderten Gasgeschwindigkeiten und den niedrigen Druckverlust in der Kolonne aus.

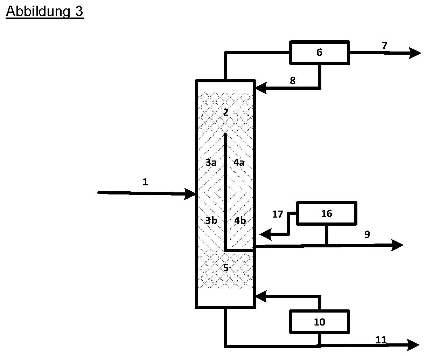

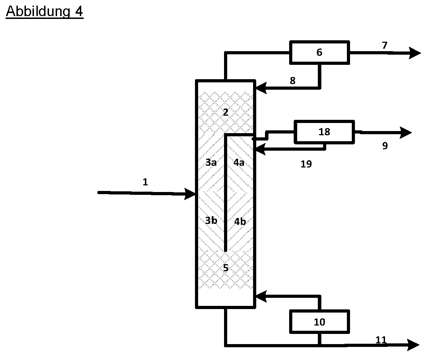

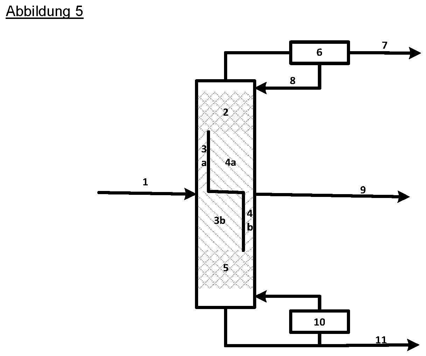

Besonders bevorzugt ist das erfindungsgemäße Verfahren ein Verfahren mit einer Trennwandkolonne mit mindestens einer Trennwand mit einer der folgenden Ausführungen:
a) Die Trennwand kann sich bevorzugt von einem trennwandlosen Sumpfbereich bis zu einem trennwandlosen Kopfbereich erstrecken. Dabei ist sowohl im Kopfbereich als auch im Sumpfbereich über den gesamten Querschnitt ein Übertritt der Einsatzstoffe möglich. Weiter bevorzugt verläuft die Trennwand durchgehend von 10 % bis 90 % der Höhe der Kolonne, jeweils gemessen vom unteren Ende der Kolonne. Weiter bevorzugt verläuft die Trennwand durchgehend von 20 und 80 % der Höhe der Kolonne, noch weiter bevorzugt von 25 bis 75 %. Die Position der Trennwand in den genannten Bereichen ermöglicht besonders gut den wirtschaftlichen Betrieb der Kolonne mit Erfüllung der hohen trenntechnischen Herausforderungen.
   Üblicherweise befindet sich die Trennwand mittig in der Kolonne. Die Trennwand kann bei dieser Ausführung auch zur Erzielung vorteilhafter Eigenschaften einen Versatz in Richtung des Zulaufs oder in Richtung der Seitenstromentnahme aufweisen. Bevorzugt beträgt der Versatz der Trennwand einen Wert zwischen 0 und 30 % des Durchmessers von der Mitte in Richtung des Seitenstrombereichs (Bereiche 4a, 4b in Abbildung 1) oder des Zulaufbereichs (Bereiche 3a, 3b in Abbildung 1). Weiter bevorzugt beträgt dieser Wert zwischen 0 und 20 %.
   Der Zulauf befindet sich weiterhin bevorzugt sich auf der einen Seite der Trennwand in einer Höhe von 25 und 66 % der Höhe der Kolonne, gemessen vom unteren Ende derselben. Weiter bevorzugt befinden sich ober- und unterhalb des Zulaufs zwischen 2 und 50 theoretische Trennstufen getrennt von der anderen Seite der Trennwand.
   Der Seitenstrom wird bei dieser Ausführung bevorzugt in einer Höhe von 30 und 60 %, weiter bevorzugt in einer Höhe von 40 bis 60 %, noch weiter bevorzugt in einer Höhe von 45 bis 55 % der Kolonnenhöhe, gemessen vom unteren Ende, auf der anderen Seite der Trennwand abgenommen. Die so ausgeführte Trennwandkolonne löst besonders gut die vom Fachmann nicht erwartete Aufreinigung von Methylenbis(cyclohexylamin) unter den angegebenen Gesichtspunkten der trenntechnischen Herausforderungen und energetischen Anforderungen des erforderlichen Druckverlusts, bei besonders niedrigen Temperaturen und unter Erzielung einer besonders hohen Reinheit. Leicht-, Mittel- und Schwersieder können bei dieser Verschaltung nur mit jeweils einem eingesetzten Kondensator und einem Verdampfer getrennt werden.
b) Die Trennwand kann zur Erzielung vorteilhafter Ergebnisse auch so ausgestaltet werden, dass die Trennwand das untere Ende der Kolonne verschließt, aber nicht den Kopfbereich. Bevorzugt verschließt dabei die Trennwand den auf der Seite der Seitenstromabnahme liegenden Teil der Kolonne (Abbildung 3) oder erstreckt sich bis zum Boden des Sumpfes.
   Üblicherweise befindet sich die Trennwand mittig in der Kolonne. Die Trennwand kann bei dieser Ausführung auch zur Erzielung vorteilhafter Eigenschaften einen Versatz in Richtung des Zulaufs und in Richtung der Seitenstromentnahme aufweisen. Bevorzugt beträgt der Versatz der Trennwand einen Wert zwischen 0 und 30 % des Durchmessers von der Mitte in Richtung des Seitenstrombereichs oder des Zulaufbereichs. Weiter bevorzugt beträgt dieser Wert zwischen 0 und 20 %.
   Der Zulauf befindet sich weiterhin bevorzugt sich auf der einen Seite der Trennwand in einer Höhe von 25 und 66 % der Höhe der Kolonne, gemessen vom unteren Ende derselben. Weiter bevorzugt befinden sich ober- und unterhalb des Zulaufs zwischen 2 und 50 theoretische Trennstufen getrennt von der anderen Seite der Trennwand.
   Der Seitenstrom wird bei dieser Ausführung weiter bevorzugt in einer Höhe von 30 und 60 % der Kolonnenhöhe, gemessen vom unteren Ende, auf der anderen Seite der Trennwand abgenommen. Die Abnahme des Seitenstroms erfolgt bevorzugt auf der unteren Stufe des Seitenstrombereichs der Trennwand als Komplettabnahme. Weiter bevorzugt wird ein Teil des Stroms erneut durch einen Verdampfer in die Gasphase überführt und auf der gleichen Stufe wie die Seitenstromabnahme wieder eingeleitet.
   Bei der komplett bis nach unten durchgezogenen Trennwand resultieren zwei unabhängige Sümpfe. Bevorzugt wird die Sumpfausschleusung mit zwei Verdampfern und Trennstufen, die bei a) unterhalb der Trennwand über den gesamten Kolonnendurchmesser vorhanden sind, in gleicher Anzahl in beiden Seiten umgesetzt. Der Seitenstrom wird in diesem Fall als zweiter Sumpfstrom abgenommen.
c) Die Trennwand kann zur Erzielung vorteilhafter Ergebnisse auch so ausgestaltet werden, dass die Trennwand den Kopfbereich verschließt, aber nicht das untere Ende der Kolonne. Bevorzugt stellt dabei die Trennwand eine Sperrwand zum trennwandlosen Kopfbereich (Abbildung 4) oder eine bis zum Kopf durchgezogene Trennwand dar. Die Veränderungen stellen sich auch bei diesem Konzept ähnlich wie bei b) dar. Der Seitenstrom wird bevorzugt allerdings nicht als zweiter Sumpfstrom oder flüssig oberhalb der Sperrwand abgenommen, sondern als zweites Destillat oder kondensiert unterhalb der Sperrwand. Bei diesem Konzept ist ein zweiter Kondensator notwendig. Ein Vorteil besteht darin, dass oberhalb der Trennwand keine Leichtsieder in den Seitenstrombereich der Trennwandkolonne übertreten können und den Seitenstrom verunreinigen können.
d) Die Trennwand kann zur Erzielung vorteilhafter Ergebnisse auch als asymmetrische Trennwand ausgestaltet sein (Abbildung 5). Eine asymmetrisch ausgestaltete Trennwand weist einen horizontalen Versatz auf. Weiter bevorzugt ist hierbei der horizontale Versatz so ausgestaltet, dass das untere Ende der Trennwand sich näher an der Seitenstromentnahme befindet als das obere Ende. Dadurch verringert sich dort die Querschnittfläche unterhalb der Seitenstromentnahme, während sie im oberen Bereich auf der Seite des Zulaufs erhöht ist. Der Vorteil dieses Aufbaus besteht darin, dass die Flüssigkeitsbelastung und die Gasverteilung und damit der Druckverlust der Kolonne optimiert wird. Weiter bevorzugt beträgt der Versatz der Trennwand oberhalb des Zulaufs 2 bis 30 % der Querschnittsfläche der Kolonne und der Versatz der Trennwand unterhalb des Seitenstrom zwischen 2 und 40 % der Querschnittsfläche der Kolonne. So kann ein besonders positiver Effekt in Bezug auf die Qualitäts-, Ausbeute- und Energieanforderungen erzeugt werden.

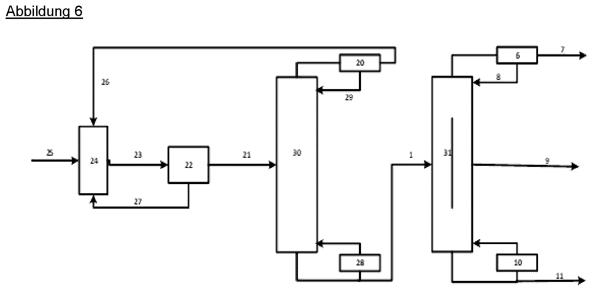

Abbildung 6 zeigt einen bevorzugten schematischen Aufbau des gesamten Verfahrens, beginnend mit der Reaktion (24), nachfolgender Katalysatorabtrennung (22), Lösemittelabtrennung (30) und nachfolgender Aufreinigung in der Trennwandkolonne (31).

### Beispiele

### Beispiel 1:

Um die Durchführbarkeit der Trennung in einer Trennwandkolonne zu zeigen, wurden Experimente im Pilotmaßstab durchgeführt. Dazu wurde eine Kolonne mit einem Durchmesser von 150 mm, die sich im mittleren Bereich in zwei Kolonnen mit Durchmessern von je 100 mm aufteilte, betrieben. Die Kolonne hatte eine Gesamthöhe von 19 Metern, wobei 9 Meter davon mit einer Gewebepackung mit 500 m²/m³ gefüllt wurden. Der Sumpf bestand aus Metall und umschloss einen Sumpfkreislauf und einen Verdampfer (10).

Die auf dem Sumpf aufbauende Kolonne bestand aus Glas. Auf der Glaskolonne baute ein Metallkondensator (6) auf.

Die Glaskolonne war in vier Bereiche unterteilt: im oberen Bereich (2) hatte die Kolonne zwei Meter Gewebepackung, im Zulaufbereich (3a und 3b) vier Meter Gewebepackung (der Zulauf wurde in der Mitte des Zulaufbereichs aufgegeben), im Seitenstrombereich vier Meter Packung (4a und 4b) und im unteren Bereich (5) zwei Meter Gewebepackung. Das Destillat wurde zum Teil als Rücklauf in die Kolonne zurückgeschickt (8) und zum Teil als Leichtsiederstrom ausgetragen (7). Der Seitenstrom (9) wurde in der Mitte von Bereich 4 abgenommen. Der Sumpfstrom (11) wurde aus dem Sumpfkreislauf abgenommen.

### Durchführung:

Methylenbis(cyclohexylamin) aus der Hydrierung von MDA wurde nach der Lösemittel-Abtrennung mit o. g. Aufbau aufgetrennt (siehe auch Abbildung 1). Dazu wurden in Pilotversuchen an einer bestehenden Kolonne 12 kg/h Methylenbis(cyclohexylamin) auf die Mitte des Vorfraktionierungsbereichs (3a und 3b) gegeben. Die Temperatur des flüssigen Zulaufs betrug dabei 170 °C. Der Kopfdruck der Kolonne wurde auf 7 bis 10 mbar eingestellt. Der kondensierte Brüden wurde zum Teil als Destillataustrag ausgeschleust und zum anderen Teil auf die Kolonne zurückgeschickt als Rücklauf. Die Flüssigkeitsmenge, die oberhalb der Trennwand anfällt, wurde in einem Verhältnis von 25 % (Zulaufbereich, oberhalb 3a) und 75 % (Seitenstrombereich, oberhalb 4a) zwischen beiden Seiten verteilt. Auf der Mitte des Seitenstrombereichs (4a, 4b) wird das aufgereinigte Methylenbis(cyclohexylamin) in Reinheiten oberhalb von 99,3 % abgenommen.

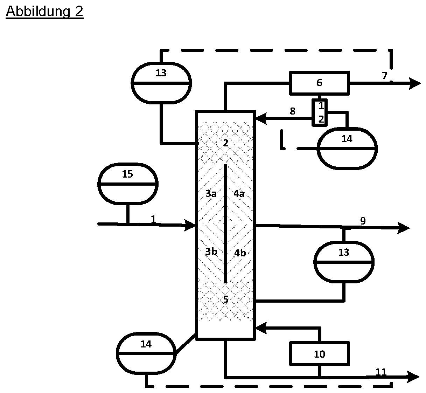

Der Sumpfstrom wurde aus dem Sumpfkreislauf genommen, sodass die Verluste an Methylenbis(cyclohexylamin) minimiert werden. Die Regelung der Produktströme und deren Reinheiten erfolgen über ein Regelkonzept (Abbildung 2), welches über Füllstand-, Temperatur und Durchflussregelungen die Ströme passend einregelt.

### Beispiel 2:

Als eine Alternative zu den Versuchsbedingungen im vorherigen Versuchspunkt, wurde die Kolonne mit einem Flüssigkeitssplit von 40 % Zulaufbereich (3a, 3b) und 60 % Seitenstrombereich (4a, 4b) betrieben. Bei veränderten Zulaufzusammensetzungen ändern sich die Anforderungen an den Flüssigkeitssplit zwischen beiden Seiten und dieser muss verändert werden, um eine optimale Trennung unter Berücksichtigung des energetischen Optimums zu gewährleisten.

### Beispiel 3:

Als zusätzliche Alternative wurde die Belastung in der Kolonne erhöht (circa 40 %) über einen höheren Zulaufstrom (16,6 kg/h) bei gleichem Flüssigkeitssplit wie in Versuchspunkt 1. Die Energiemenge konnte hier annähernd eingehalten werden.

Die Ergebnisse haben gezeigt, dass eine hohe Reinheit an Methylenbis(cyclohexylamin) erreicht werden kann und gleichzeitig die Ausbeute sehr hoch ist, bei kleinen Verlusten in den Sumpf- und Destillatströmen.

Durch den Aufbau als Glaskolonne konnte während und nach den Versuchen in die Kolonne geschaut werden und es wurden keine Verunreinigungen festgestellt.

Die nachfolgend abgebildete Tabelle 1 fasst die Betriebsparameter und Ergebnisse der Beispiele 1 - 3 zusammen:

**Tabelle 1:**

| Nr. | - | 1 | 2 | 3 |
|---|---|---|---|---|
| Kopfdruck | mbar,a | 7 | 9 | 8 |
| Sumpfdruck | mbar,a | 13 | 18 | 16 |
| Kopf-Temperatur | °C | 127,5 | 140,7 | 138,5 |
| Sumpf-Temperatur | °C | 223,6 | 225,5 | 226,2 |
| Flüssigkeitssplit | - | 25/75 | 40/60 | 25/75 |
| Rücklauf | kg/h | 13,69 | 21,39 | 18,87 |
| Zulauf-Temperatur | °C | 170 | 170 | 170 |
| Zulauf | kg/h | 11,94 | 11,95 | 16,63 |
| Destillat | kg/h | 0,11 | 0,13 | 0,18 |
| Seitenstrom | kg/h | 10,00 | 9,91 | 13,94 |
| Sumpf | kg/h | 1,83 | 1,91 | 2,51 |
| Methylenbis(cyclohexylamin) im Destillat | wt-% | 11,92 | 20,84 | 22,07 |
| Methylenbis(cyclohexylamin) im Seitenstrom | wt-% | 99.49 | 99.43 | 99.09 |
| Methylenbis(cyclohexylamin) im Sumpf | wt-% | 6,77 | 11,85 | 9,20 |
| Energie pro kg Methylenbis(cyclohexylamin) im Seitenstrom | kJ/kg | 461 | 763 | 491 |

### Bezugszeichenliste:

1 - Zulauf
2 - oberer Bereich/Kopf
3 - Zulaufbereich, Vorfraktionierungsbereich, a oberhalb des Zulaufs, b unterhalb des Zulaufs
4 - Seitenstrombereich, a oberhalb der Seitenstromabnahme, b unterhalb der Seitenstromabnahme
5 - unterer Bereich/Sumpf
6 - Kondensator
7 - Leichtsiederstrom
8 - Rücklauf
9 - Seitenstromentnahme
10 - Verdampfer
11 - Sumpfstrom
12 - Destillatbehälter
13 - Temperaturregelung
14 - Füllstandsregelung
15 - Durchflussregelung
16 - Seitenstromverdampfer
17 - Seitenstromverdampfer-Rückführung
18 - Seitenstromkondensator
19 - Seitenstrom-Rücklauf
20 - Kondensator Lösemittel Kolonne
21 - Zulauf Lösemittel Kolonne
22 - Katalysator Abscheider
23 - Reaktionsprodukt
24 - Reaktor
25 - Reaktions-Edukte
26 - Lösemittel-Rückführung
27 - Katalysator Rückführung
28 - Verdampfer Lösemittel Kolonne
29 - Rücklauf Lösemittel Kolonne
30 - Lösemittelabtrennung (Kolonne)
31 - Trennwandkolonne

## Patentansprüche

1. Verfahren zur Herstellung von Methylenbis(cyclohexylamin) umfassend
1) katalytische Hydrierung von MDA,
2) Abtrennung des Katalysators und
3) nachfolgende Destillation des Produktes der Hydrierung,
**dadurch gekennzeichnet, dass**
bei der Destillation in Schritt 3) mindestens eine Trennwandkolonne eingesetzt wird.

2. Verfahren nach Anspruch 1,
**dadurch gekennzeichnet, dass**
- als Katalysator in Schritt 1) ein geträgerter Katalysator eingesetzt wird,
- der Aktivmetall in einer Menge von 0,01 bis 20 Gew.-%, bezogen auf den geträgerten Katalysator, auf einen Träger aufgebracht enthält,
- und dessen Aktivmetall Ruthenium alleine oder Ruthenium und mindestens ein Metall der I., VII. oder VIII. Nebengruppe des Periodensystems ist.

3. Verfahren nach Anspruch 1 oder 2,
**dadurch gekennzeichnet, dass**
das eingesetzte MDA mindestens 70 Gew.-% 4,4'-Diaminodiphenylmethan und 0,01 bis 2 Gew.-% N-Methylverbindungen, jeweils bezogen auf die Gesamtmasse an Verbindungen mit aromatischen Ringen, aufweist.

4. Verfahren nach einem der vorhergehenden Ansprüche,
**dadurch gekennzeichnet, dass**
die Destillation des Produktes der Hydrierung in Schritt 3) ausschließlich durch Einsatz einer Trennwandkolonne erfolgt.

5. Verfahren nach einem der vorhergehenden Ansprüche,
**dadurch gekennzeichnet, dass**
sich die Trennwand der mindestens einen Trennwandkolonne durchgehend in einer Höhe von 15 bis 80 % der Trennwandkolonne, gemessen vom unteren Ende, befindet.

6. Verfahren nach einem der vorhergehenden Ansprüche,
**dadurch gekennzeichnet, dass**
die Trennwand der mindestens einen Trennwandkolonne einen Versatz von 0 bis 30 % des Durchmessers von der Mitte in Richtung des Seitenstrombereichs oder des Zulaufbereichs aufweist.

7. Verfahren nach einem der vorhergehenden Ansprüche,
**dadurch gekennzeichnet, dass**
die Trennwand der mindestens einen Trennwandkolonne als asymmetrische Trennwand ausgestaltet ist.

8. Verfahren nach einem der vorhergehenden Ansprüche,
**dadurch gekennzeichnet, dass**
die Destillation mit mindestens einer Trennwandkolonne in Schritt 3) so betrieben wird, dass
a) sich auf der Höhe mindestens eines Teils der Trennwand der Zulauf befindet,
b) sich auf der Höhe mindestens eines Teils der Trennwand auf der anderen Seite der Trennwand, bezogen auf den Zulauf, die Seitenstromentnahme zur Entnahme von aufgereinigtem Methylenbis(cyclohexylamin) befindet,
c) am Kopf der Trennwandkolonne das Destillat entnommen, und zum Teil ausgetragen und zum Teil wieder in die Trennwandkolonne zurückgeführt wird und
d) der am Boden der Trennwandkolonne befindliche Sumpf ausgeschleust wird.

9. Verfahren nach Anspruch 8,
**dadurch gekennzeichnet, dass**
die Entnahme des Destillats in Schritt c) so ausgeführt wird, dass
- am Kopf der Trennwandkolonne das Destillat entnommen,
- es in einem angeschlossenen Kondensator mindestens partiell kondensiert,
- und mindestens ein Teil des Kondensats in den Kopfbereich der Trennwandkolonne zurückgeführt und ein ggf. hinter dem Kondensator noch flüchtig vorliegender Leichtsiederstrom zusammen mit nicht rückgeführtem Kondensat ausgetragen wird.

10. Verfahren nach einem der vorhergehenden Ansprüche,
**dadurch gekennzeichnet, dass**
sich der Zulauf der mindestens einen Trennwandkolonne in einer Höhe von 25 bis 66 % der Höhe der Kolonne, gemessen vom unteren Ende befindet.

11. Verfahren nach einem der vorhergehenden Ansprüche,
**dadurch gekennzeichnet, dass**
die mindestens eine Trennwandkolonne eine Zahl theoretischer Trennstufen von 10 bis 90 aufweist.

12. Verfahren nach einem der vorhergehenden Ansprüche,
**dadurch gekennzeichnet, dass**
die mindestens eine Trennwandkolonne bei einer Sumpftemperatur von 190 bis 300 °C betrieben wird.

13. Verfahren nach einem der vorhergehenden Ansprüche,
**dadurch gekennzeichnet, dass**
die Flüssigkeitsbelastung unterhalb der Seitenstromabnahme und oberhalb des Sumpfes und oberhalb des unteren Endes der Trennwand kleiner oder gleich 1,5 m³/(m²h) ist.

14. Verfahren nach einem der vorhergehenden Ansprüche,
**dadurch gekennzeichnet, dass**
sich die Seitenstromentnahme der mindestens einen Trennwandkolonne in einer Höhe von 30 bis 60% der Kolonne, gemessen vom unteren Ende, befindet.

15. Verfahren nach einem der vorhergehenden Ansprüche,
**dadurch gekennzeichnet, dass**
die Packungsbetten der mindestens einen Trennwandkolonne eine Höhe von 2 bis 7 Metern aufweisen.
